# EUROPEAN PATENT APPLICATION

(11) **EP 2 060 564 A1**
(43) Date of publication of application: **20.05.2009**
(21) Application number: 07120988.6
(22) Date of filing: 19.11.2007
(51) Int. Cl.: C07D 211/58, A61K 31/4468

(54) **Non-peptidic promoters of apoptosis**

(71) Applicant: Ludwig-Maximilians-Universität München, 80539 München (DE); Leopold-Franzens-Universität Innsbruck, 6020 Innsbruck (AT)
(72) Inventor: Stuppner, Hermann, 6091 Götzens (AT); Langer, Thierry, 6094 Axams (AT); Rollinger, Judith, 6020 Innsbruck (AT); Bliem, Caroline, 6020 Innsbruck (AT); Vollmar, Angelika, 82049 Pullach i. Isartal (DE); Barth, Nicole, 81375 München (DE); Schyschka, Lillianna, 85354 Freising (DE)
(74) Representative: Vossius & Partner

(57) **Abstract**

The present invention relates to the use of two classes of non-peptidic compounds as medicaments. In particular, these compounds can be used for the treatment of hyperproliferative diseases.

## Description

The present invention relates to the use of compounds of formulae (I) and (II) as defined herein as medicaments. In particular, these compounds can be used for the treatment of hyperproliferative diseases.

Apoptosis is a genetically programmed process of cell death that allows for the removal of excess or damaged cells. In fact, the network of apoptotic signalling mechanism in most cell types provides a major barrier to the development and progression of human cancer. Most commonly used radiation and chemotherapies rely on activation of apoptotic pathways to kill tumor cells and, importantly, tumor cells which are capable of evading programmed cell death often become resistant to treatment. Apoptotic signalling involves a specific cascade of cysteine proteases, termed caspases, whose activity is initiated by the conversion of a zymogen form to an active form. The subsequent cleavage of caspase substrates results in the disassembly of the cell. Caspases function within the cells in a hierarchical order involving initiator caspases (e.g. caspasae-8 for the extrinsic pathway and caspase-9 for the intrinsic pathway) that cleave effector caspases (e.g. caspase-3).

Regulation of caspase activation and activity is provided by members of the inhibitor of apoptosis protein (IAP) family. IAPs sabotage apoptosis by directly interacting with and neutralizing caspases (Wrzesien-Kus et al., Apoptosis (2004), 9(6), 705-715). Their deregulation in a variety of pathologies especially in cancer and furthermore chemoresistance has placed them in the focus as potential targets for drug discovery (Devi, Drug News Perspect. (2004), 17(2), 127-134; Schimmer et al., Hematology Am. Soc. Hematol. Educ. Program (2005) 215-219; Schimmer et al., Cell Death Differ. (2006), 13(2), 179-188). XIAP is the prototype IAP and has three functional domains referred to as BIR 1, BIR 2 and BIR 3. BIR 3 interacts directly with caspase-9 and inhibits its ability to bind and cleave procaspase-3, its natural substrate. BIR 2 interacts directly with caspase-3. In each case, however, inhibition of the caspase can be relieved by the binding of the proapoptotic mitochondrial protein SMAC, also known as DIABLO (Wrzesien-Kus et al., Apoptosis (2004), 9(6), 705-715; Arkin, Curr. Opin. Chem. Biol. (2005), 9(3), 317-324).

It is an object of the present invention to provide compounds that can be used for the treatment of hyperproliferative diseases, such as tumorous diseases, non-solid cancers and metastases. A particular object of the present invention is to overcome chemoresistance upon treatment with conventional chemotherapeutics (e.g. etoposide or taxol) or experimental therapeutics, such as TRAIL, which are for example described by Li et al., Science (2004), 305(5689), 1471-1474; and Wrzesien-Kus et al., Apoptosis (2004), 9(6), 705-715. A further object of the present invention is the provision of compounds having the above activities which can readily be synthesized. Yet another object of the present invention is the provision of compounds having the above activities which are of low toxicity.

The present inventors surprisingly found that these objects can be achieved by compounds of formula (I) and compounds of formula (II) as defined hereinafter.

Thus, the present invention relates in a first aspect to a compound of formula (I) 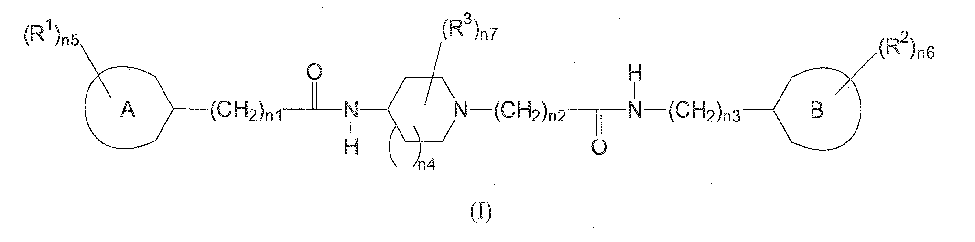 or a pharmaceutically acceptable salt thereof, a pharmaceutically acceptable solvate thereof or a pharmaceutically acceptable prodrug thereof for use as a medicament.

A is a saturated, unsaturated or aromatic 5 to 10-membered mono- or bicyclic ring system, which optionally contains one or more heteroatoms, selected from N, O and S. Preferably, A is a saturated or aromatic 6-membered carbocyclic ring, more preferably a phenyl ring.

B is a saturated, unsaturated or aromatic 5 to 10-membered mono- or bicyclic ring system, which optionally contains one or more heteroatoms, selected from N, O and S. Preferably, B is an aromatic 6-membered carbocyclic ring, such as a phenyl ring.

Each R¹ is independently selected from C₁₋₄ alkyl, C₁₋₄ alkoxy, C₆₋₁₀ aryl, SO₂NH-C₁₋₄ alkyl, SO₂NH-C₆₋₁₀ aryl and halogen. Preferably, each R¹ is independently selected from C₁₋₄ alkyl, C₁₋₄ alkoxy and halogen (preferably Cl or Br).

Each R² is independently selected from C₁₋₄ alkyl, C₁₋₄ alkoxy, C₆₋₁₀ aryl, SO₂NH-C₁₋₄ alkyl, SO₂NH-C₆₋₁₀ aryl and halogen. Preferably, each R² is independently selected from C₁₋₄ alkyl, C₁₋₄ alkoxy and halogen (preferably Cl or Br).

Each R³ is independently selected from C₁₋₄ alkyl, C₁₋₄ alkoxy, C₆₋₁₀ aryl, SO₂NH-C₁₋₄ alkyl, SO₂NH-C₆₋₁₀ aryl and halogen, or wherein two R³, which are bonded to neighboring carbon atoms, can be combined to form, together with the carbon atoms to which they are bonded, a fused 6-membered aromatic ring.

In the above definitions of R¹, R² and R³ one or more carbon atoms in the aryl groups may optionally be replaced by a heteroatom selected from N, O and S. The resulting heteroaryl groups include pyrrolidinyl, tetrahydrofuranyl, tetrahydrothiophenyl, piperidinyl, pyrrolyl, furanyl, thiophenyl, pyridinyl, pyridazinyl, pyrimidinyl, pyrazinyl, triazinyl, tetrazinyl, quinolinyl, indenyl, oxazolyl, thiazolyl, imidazolyl, pyrazolyl, isoxazolyl, isothiazolyl, oxadiazolyl, triazolyl and thiadiazolyl. In the SO₂NH-heteroaryl groups, the SO₂NH moiety is bonded to a carbon atom of the heteroaryl group. n1 is an integer from 0 to 3. Preferably, n1 is 0 or 1. In one preferred aspect, n1 is 0. In another preferred aspect, n1 is 1. n2 is an integer from 0 to 3. Preferably, n2 is 1 or 2, more preferably n2 is 1. n3 is an integer from 0 to 3. Preferably, n3 is 0 or 1, more preferably n3 is 0. n4 is an integer from 0 to 2. Preferably, n4 is 1. n5 is an integer from 0 to 3. In one preferred aspect, n5 is 0. In another preferred aspect, n5 is 1. n6 is an integer from 0 to 5. In one preferred aspect, n6 is 0. In another preferred aspect, n6 is 1.
In yet another preferred aspect, n6 is 2. In yet another preferred aspect, n6 is 3. n7 is an integer from 0 to 3. Preferably, n7 is 0.

The following compounds of formulae (I-1) to (I-11) are employed in particularly preferred embodiments. 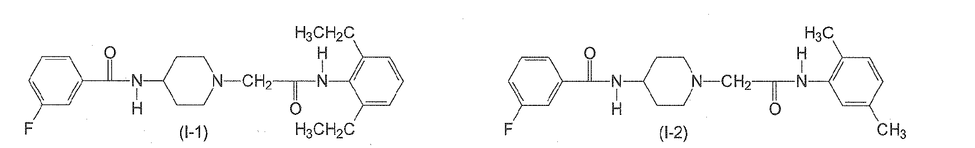 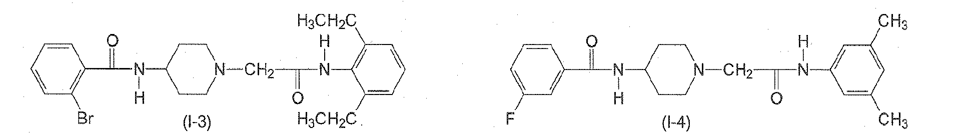 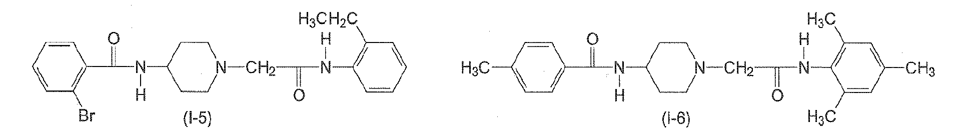 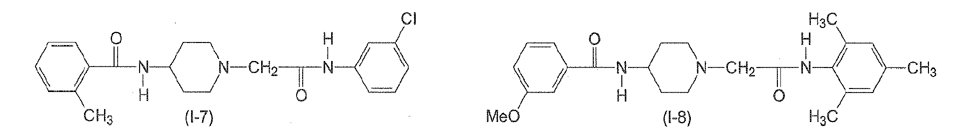 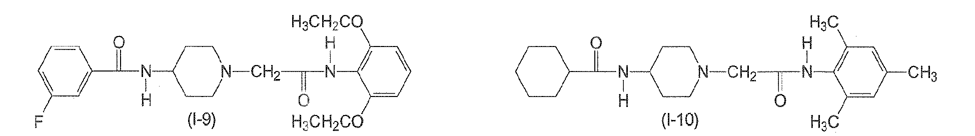 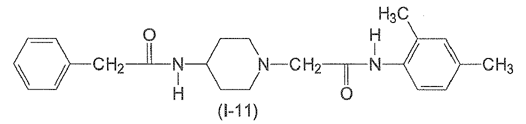 Among compounds of formulae (I-1) to (I-11), the compounds of formulae (I-1), (I-3), (I-9) and (I-11) are particularly preferred and the compound of formula (I-11) is even more preferred.

In a further aspect, the present invention relates to a compound of formula (II) 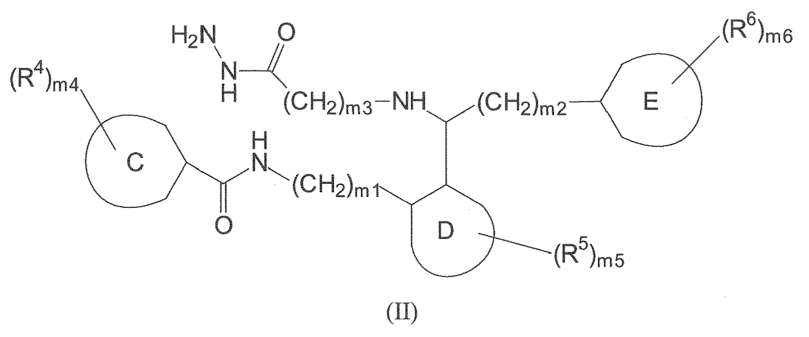 or a pharmaceutically acceptable salt thereof, a pharmaceutically acceptable solvate thereof or a pharmaceutically acceptable prodrug thereof for use as a medicament.

C is a saturated, unsaturated or aromatic 5 to 10-membered mono- or bicyclic ring system, which optionally contains one or more heteroatoms, selected from N, O and S. Preferably, C is a 6-membered carbocyclic ring, more preferably an aromatic 6-membered carbocyclic ring.

D is a saturated, unsaturated or aromatic 5 to 10-membered mono- or bicyclic ring system, which optionally contains one or more heteroatoms, selected from N, O and S. Preferably, D is a 6-membered carbocyclic ring, more preferably an aromatic 6-membered carbocyclic ring.

E is a saturated, unsaturated or aromatic 5 to 10-membered mono- or bicyclic ring system, which optionally contains one or more heteroatoms, selected from N, O and S. Preferably, E is a 6-membered carbocyclic ring, more preferably an aromatic 6-membered carbocyclic ring.

Each R⁴ is independently selected from C₁₋₄ alkyl, C₁₋₄ alkoxy, C₆₋₁₀ aryl, SO₂NH-C₁₋₄ alkyl, SO₂NH-C₆₋₁₀ aryl and halogen. Preferably, each R⁴ is independently selected from C₁₋₄ alkyl, C₁₋₄ alkoxy and halogen (preferably Cl or Br). More preferably, each R⁴ is Br.

Each R⁵ is independently selected from C₁₋₄ alkyl, C₁₋₄ alkoxy, C₆₋₁₀ aryl, SO₂NH-C₁₋₄ alkyl, SO₂NH-C₆₋₁₀ aryl and halogen. Preferably, each R⁵ is independently selected from C₁₋₄ alkyl, C₁₋₄ alkoxy and halogen (preferably Cl or Br). More preferably, each R⁵ is Br.

Each R⁶ is independently selected from C₁₋₄ alkyl, C₁₋₄ alkoxy, C₆₋₁₀ aryl, SO₂NH-C₁₋₄ alkyl, SO₂NH-C₆₋₁₀ aryl and halogen. Preferably, each R⁶ is independently selected from C₁₋₄ alkyl, C₁₋₄ alkoxy and halogen (preferably Cl or Br).
m1 is an integer from 0 to 2, preferably 0.
m2 is an integer from 0 to 2, preferably 0.
m3 is an integer from 1 to 3, preferably 1.
m4 is an integer from 0 to 2, preferably 0 or 1. In a preferred embodiment, m4 is 1 and R⁴ is Br.
m5 is an integer from 0 to 3, preferably 0 or 1 In a preferred embodiment, m5 is 1 and R⁵ is Br.
m6 is an integer from 0 to 3, preferably 0 or 1.

In the above definitions of R⁴, R⁵ and R⁶ one or more carbon atoms in the aryl groups may optionally be replaced by a heteroatom selected from N, O and S. The resulting heteroaryl groups include pyrrolidinyl, tetrahydrofuranyl, tetrahydrothiophenyl, piperidinyl, pyrrolyl, furanyl, thiophenyl, pyridinyl, pyridazinyl, pyrimidinyl, pyrazinyl, triazinyl, tetrazinyl, quinolinyl, indenyl, oxazolyl, thiazolyl, imidazolyl, pyrazolyl, isoxazolyl, isothiazolyl, oxadiazolyl, triazolyl and thiadiazolyl. In the SO₂NH-heteroaryl groups, the SO₂NH moiety is bonded to a carbon atom of the heteroaryl group.

The following compound of formula (II-1) is employed in particularly preferred embodiment.

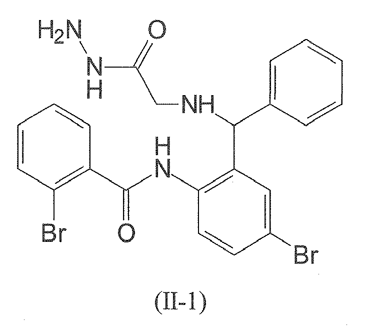

Compounds falling under formulae (I) and (II) are commercially available, for example from Asinex Europe B.V. (Rijswijk, The Netherlands). Further, a skilled chemist will be readily in a position to devise synthetic routes to compounds falling under formulae (I) and (II).

Compounds of formula (I) can, for example, be obtained by the following synthetic route, involving two successive peptide couplings:

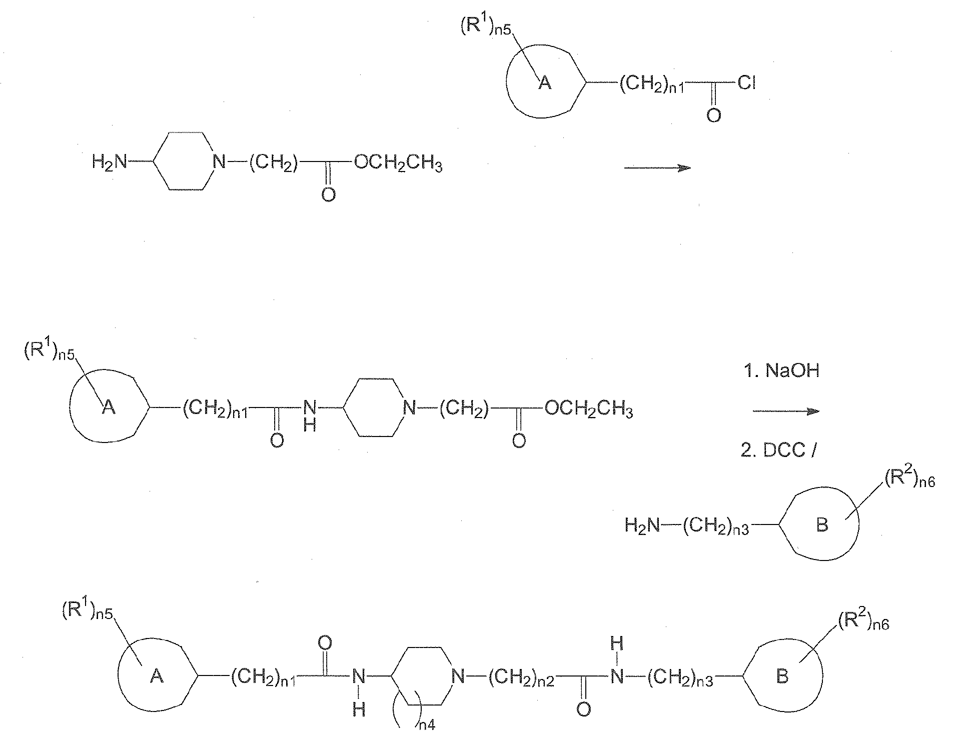

DCC stands for N,N'-dicyclohexyl-carbodilmide. The synthesis of the aminopiperdine used as a starting material is, for example, described in Walker et al., J. Org. Chem. (1961) 26, 2740.

Compounds of formula (II) can, for example, be obtained by the following synthetic route, involving a peptide coupling followed by a reductive amination:

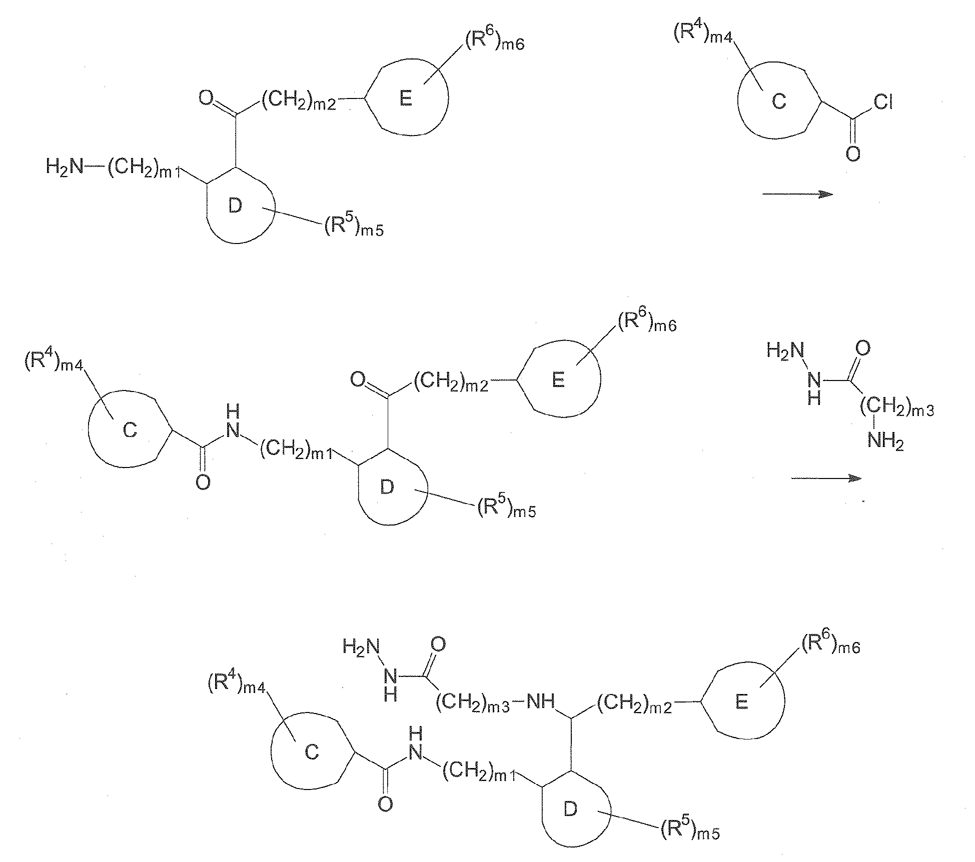

Depending on the type of substitution in the target compounds falling under formulae (I) and (II), the skilled person will employ suitable protection group chemistry, for example based on the principles laid out in P. G. M. Wuts, T. W. Greene "Greene's Protective Groups in Organic Synthesis" Wiley & Sons, 4th edition (2006).

Pharmaceutically acceptable salts of compounds that can be used in the present invention can be formed with various organic and inorganic acids and bases. Exemplary base addition salts comprise, for example, alkali metal salts such as sodium or potassium salts; alkaline-earth metal salts such as calcium or magnesium salts; ammonium salts; aliphatic amine salts such as trimethylamine, triethylamine, dicyclohexylamine, ethanolamine, diethanolamine, triethanolamine, procaine salts, megluinine salts, diethanol amine salts or ethylenediamine salts; aralkyl amine salts such as N, N-dibenzylethylenediamine salts, benetamine salts; heterocyclic aromatic amine salts such as pyridin salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts or lysine salts. Exemplary acid addition salts comprise, for example, mineral acid salts such as hydrochloride, sulfate salts, nitrate salts, phosphate salts, carbonate salts, hydrogencarbonate salts or perchlorate salts; organic acid salts such as acetate, propionate, lactate, maleate, fumarate, tararic acid salt, malate, citrate or ascorbates salts; sulfonate salts such as methanesulfonate, benzenesulfonate, or p-toluenesulfonate salts; and acidic amino acid salts such as aspartate or glutamate salts.

Pharmaceutically acceptable solvates of compounds that can be used in the present invention may exist in the form of solvates with water, for example hydrates, or with organic solvents such as methanol, ethanol or acetonitrile, i.e. as a methanolate, ethanolate or acetonitrilate, respectively.

Pharmaceutically acceptable prodrugs of compounds that can be used in the present invention are derivatives which have chemically or metabolically cleavable groups and become, by solvolysis or under physiological conditions, the compounds used in the present invention which are pharmaceutically active in vivo. Prodrugs of compounds that can be used in the present invention may be formed in a conventional manner with a functional group of the compounds such as with an amino, hydroxy or carboxy group. The prodrug derivative form often offers advantages of solubility, tissue compatibility or delayed release in a mammalian organism (see, Bundgaard, H., Design of Prodrugs, pp. 7-9, 21-24, Elsevier, Amsterdam 1985).

Prodrugs include acid derivatives well known to the person skilled in the art, such as, for example, esters prepared by reaction of the parent acidic compound with a suitable alcohol, or amides prepared by reaction of the parent acid compound with a suitable amine. When a compound employed in the present invention has a carboxyl group, an ester derivative prepared by reacting the carboxyl group with a suitable alcohol or an amide derivative prepared by reacting the carboxyl group with a suitable amine is exemplified as a prodrug. An especially preferred ester derivative as an prodrug is methylester, ethylester, n-propylester, isopropylester, n-butylester, isobutylester, tert-butylester, morpholinoethylester or N,N-diethylglycolamidoester. When a compound employed in the present invention has a hydroxy group, an acyloxy derivative prepared by reacting the hydroxyl group with a suitable acylhalide or a suitable acid anhydride is exemplified as a prodrug. An especially preferred acyloxy derivative as a prodrug is -O(=O)-CH₃, -OC(=O)-C₂H₅, -OC(=O)-(tert-Bu), -OC(=O)-C₁₅H₃₁, -OC(=O)-(m-COONa-Ph), -OC(=O)-CH₂CH₂COONa, -O(C=O)-CH(NH₂)CH₃ or -OC(=O)-CH₂-N(CH₃)₂. When a compound employed in the present invention has an amino group, an amide derivative prepared by reacting the amino group with a suitable acid halide or a suitable mixed anhydride is exemplified as a prodrug. An especially preferred amide derivative as a prodrug is -NHC(=O)-(CH₂)₂OCH₃ or -NHC(=O)-CH(NH₂)CH₃.

While it is possible for the compounds of the present invention to be administered alone, it is preferable to formulate them into a pharmaceutical composition in accordance with standard pharmaceutical practice. Thus the invention also provides a pharmaceutical composition which comprises a therapeutically effective amount of a compound of formula (I) or (II) in admixture with a pharmaceutically acceptable excipient.

Pharmaceutically acceptable excipients are well known in the pharmaceutical art, and are described, for example, in Remington's Pharmaceutical Sciences, 15th Ed., Mack Publishing Co., New Jersey (1991). The pharmaceutical excipient can be selected with regard to the intended route of administration and standard pharmaceutical practice. The excipient must be acceptable in the sense of being not deleterious to the recipient thereof.

Pharmaceutically useful excipients that may be used in the formulation of the pharmaceutical composition of the present invention may comprise, for example, carriers, vehicles, diluents, solvents such as monohydric alcohols such as ethanol, isopropanol and polyhydric alcohols such as glycols and edible oils such as soybean oil, coconut oil, olive oil, safflower oil cottonseed oil, oily esters such as ethyl oleate, isopropyl myristate, binders, adjuvants, solubilizers, thickening agents, stabilizers, disintegrants, glidants, lubricating agents, buffering agents, emulsifiers, wetting agents, suspending agents, sweetening agents, colorants, flavors, coating agents, preservatives, antioxidants, processing agents, drug delivery modifiers and enhancers such as calcium phosphate, magnesium state, talc, monosaccharides, disaccharides, starch, gelatine, cellulose, methylcellulose, sodium carboxymethyl cellulose, dextrose, hydroxypropyl-β-cyclodextrin, polyvinylpyrrolidone, low melting waxes, and ion exchange resins.

The routes for administration (delivery) of the compounds employed in the invention include, but are not limited to, one or more of oral (e. g. as a tablet, capsule, or as an ingestible solution), topical, mucosal (e. g. as a nasal spray or aerosol for inhalation), nasal, parenteral (e. g. by an injectable form), gastrointestinal, intraperitoneal, intramuscular, intravenous, intrauterine, intraocular, subcutaneous, ophthalmic (including intravitreal or intracameral), rectal, buccal, and sublingual.

For example, the compounds can be administered orally in the form of tablets, capsules, ovules, elixirs, solutions or suspensions, which may contain flavoring or coloring agents, for immediate-, delayed-, modified-, sustained-, pulsed- or controlled-release applications.

The tablets may contain excipients such as microcrystalline cellulose, lactose, sodium citrate, calcium carbonate, dibasic calcium phosphate and glycine, disintegrants such as starch (preferably com, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

If the compounds of the present invention are administered parenterally, then examples of such administration include one or more of: intravenously, intraarterially, intraperitoneally, intrathecally, intraventricularly, intraurethrally, intrasternally, intracranially, intramuscularly or subcutaneously administering the compounds; and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Alternatively, the compounds of the present invention can be administered in the form of a suppository or pessary, or it may be applied topically in the form of a gel, hydrogel, lotion, solution, cream, ointment or dusting powder. The compounds of the present invention may also be dermally or transdermally administered, for example, by the use of a skin patch.

They may also be administered by the pulmonary or rectal routes. They may also be administered by the ocular route. For ophthalmic use, the compounds can be formulated as micronized suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the compounds of the present invention can be formulated as a suitable ointment containing the active compound suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, 2-octyldodecanol, benzyl alcohol and water.

Typically, a physician will determine the actual dosage which will be most suitable for an individual subject. The specific dose level and frequency of dosage for any particular individual may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

A proposed dose of the compounds according to the present invention for administration to a human (of approximately 70 kg body weight) is 0.1 mg to 1 g, preferably 1 mg to 500 mg of the active ingredient per unit dose. The unit dose may be administered, for example, 1 to 4 times per day. The dose will depend on the route of administration. It will be appreciated that it may be necessary to make routine variations to the dosage depending on the age and weight of the patient as well as the severity of the condition to be treated. The precise dose and route of administration will ultimately be at the discretion of the attendant physician or veterinarian.

The compounds employed in the present invention may be administered in combination with radiotherapy.

The compounds employed in the present invention may also be used in combination with other therapeutic agents. When the compound is used in combination with a second therapeutic agent active against the same disease the dose of each compound may differ from that when the compound is used alone.

For example, the second therapeutic agent can be an anticancer drug, such as etoposide, taxol or doxorubicine. The anticancer drug can be a tumor angiogenesis inhibitor (for example, a protease inhibitor, an epidermal growth factor receptor kinase inhibitor, or a vascular endothelial growth factor receptor kinase inhibitor); a cytotoxic drug (for example, an antimetabolite, such as purine and pyrimidine analogue antimetabolites); an antimitotic agent (for example, a microtubule stabilizing drug or an antimitotic alkaloid); a platinum coordination complex; an anti-tumor antibiotic; an alkylating agent (for example, a nitrogen mustard or a nitrosurea); an endocrine agent (for example, an adrenocorticosteroid, an androgen, an anti-androgen, an estrogen, an anti-estrogen, an aromatase inhibitor, a gonadotropin-releasing hormone agonist, or a somatostatin analogue); or a compound that targets an enzyme or receptor that is overexpressed and/or otherwise involved in a specific metabolic pathway that is misregulated in the tumor cell (for example, ATP and GYP phosphodiesterase inhibitors, histone deacetylase inhibitors, protein kinase inhibitors (such as serine, threonine and tyrosine kinase inhibitors (for example, Abelson protein tyrosine kinase)) and the various growth factors, their receptors and kinase inhibitors therefor (such as epidermal growth factor receptor kinase inhibitors, vascular endothelial growth factor receptor kinase inhibitors, fibroblast growth factor inhibitors, insulin-like growth factor receptor inhibitors and platelet-derived growth factor receptor kinase inhibitors)); methionine, aminopeptidase inhibitors, proteasome inhibitors, and cyclooxygenase inhibitors (for example, cyclooxygenase-1 or cyclooxygenase-2 inhibitors).

The combinations referred to above may conveniently be presented for use in the form of a pharmaceutical formulation. The individual components of such combinations may be administered either sequentially or simultaneously in separate or combined pharmaceutical formulations by any convenient route. When administration is sequential, either the present compound or the second therapeutic agent may be administered first. When administration is simultaneous, the combination may be administered either in the same or different pharmaceutical composition. When combined in the same formulation it will be appreciated that the two compounds must be stable and compatible with each other and the other components of the formulation. When formulated separately they may be provided in any convenient formulation, conveniently in such manner as are known for such compounds in the art.

The pharmaceutical compositions of the invention can be produced in a manner known per se to the skilled person as described, for example, in Remington's Pharmaceutical Sciences, 15th Ed., Mack Publishing Co., New Jersey (1991).

The compounds employed in the present invention can be used for treating a hyperproliferative disease, such as a tumorous disease, a non-solid cancer and/or metastases. Examples of the non-solid cancer include leukaemia or a lymphoid cancer, which can be a B-cell malignancy, such as B-cell lymphoma, non-Hodgkin lymphoma or B-cell derived chronic lymphatic leukemia (B-CLL). Examples of the hyperproliferative disease include hyperplasis, fibrosis, angiogenesis, psoriasis, atherosclerosis or smooth muscle proliferation in the blood vessels. Further hyperproliferative diseases that can be treated using the compounds employed in the present invention include hyperplasis, fibrosis (for example, pulmonary or renal fibrosis), angiogenesis, psoriasis, atherosclerosis or smooth muscle proliferation in the blood vessels (for example, stenosis or restenosis following angioplasty).

Tumorous disease that can be treated with the compounds employed in the present invention include breast cancer, genitourinary cancer (for example, a prostate tumor, especially a hormone-refractory prostate tumor), lung cancer (for example, a small cell or non-small cell lung tumor), gastrointestinal cancer (for example, a colorectal tumor), epidermoid cancer (for example, an epidermoid head and/or neck tumor or a mouth tumor), melanoma, ovarian cancer, pancreas cancer, neuroblastoma, head and/or neck cancer, bladder cancer, renal cancer, brain cancer and gastric cancer.

The compounds employed in the present invention can further be used for treating B-cell mediated autoimmune diseases, such as Myasthenia gravis, Morbus Basedow, Hashimoto thyreoiditis or Goodpasture syndrome.

In a further aspect, the present invention relates to the use of a compound of formula (I) or (II) as defined above for the preparation of a medicament for the treatment of a hyperproliferative disease. In yet a further aspect, the present invention relates to a method for treating a hyperproliferative disease, which comprises administering to a patient in need thereof an effective amount of a compound of formula (I) or (II).

Without wishing to be bound by theory, it is believed that the compounds of formulae (I) and (II) target the BIR 3 domain of XIAP, thereby promoting apoptosis. The chemical structure of the compounds of formulae (I) and (II) differ from SMAC mimitics which represent tripeptide entities as well as polyphenylureas (Schinimer, Cancer Cell (2004), 5(1), 25-35; Huang et al., Cancer Cell (2994), 5(1), 1-2; Carter et al., Blood (2005), 105(10), 4043-4050), embelin (Nikolovska-Coleska et al., J. Med. Chef. (2004), 47(10), 2430-2440; Chen et al., Bioorg. Med. Chem. Lett. (2006), 16(22), 5805-5808) or a tetrazoyl thioether C2 diyne (Li et al., Science (2004), 305(5689), 1471-1474). The compounds of formulae (I) and (II) are simple and easy to synthesize. Furthermore, they possess no significant toxicity even at high concentrations which is a major advantage regarding the therapeutical use and possible side effects. As compared to other XIAP antisense drugs which are presently in phase I/II clinical trials (Cummings et al., Br. J. Cancer (2005), 92(3), 532-538; La Casse et al., Clin. Cancer Res. (2006), 12(17), 5231-5241) the compounds of formulae (I) and (II), being small molecule inhibitors, possess advantages such as better pharmacokinetic profiles in general.

The invention will now be described by reference to the following examples which are merely illustrative and are not to be construed as a limitation of the scope of the present invention.

The invention is also illustrated by the following illustrative figures. The appended figures show:

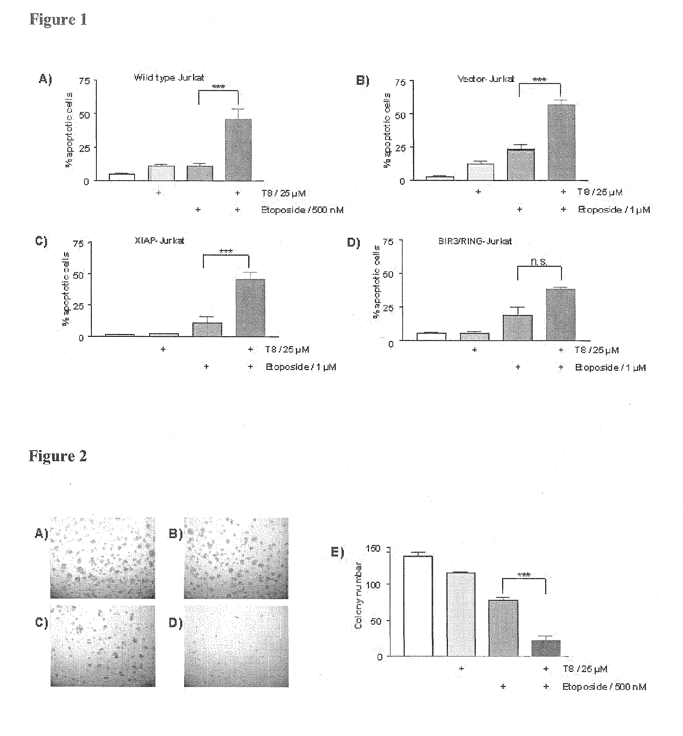

**Figure 1**: Compound I-1 sensitizes different Jurkat cell lines towards etoposide-induced apoptosis. Compound I-1 was tested in wildtype human leukemia Jurkat T cells (**A**), as well as in Jurkat cells stably transfected with empty vector (**B**), full-length XIAP overexpressing Jurkat cells (**C**) or the BtR3/Rmg domain overexpressing Jurkat cells (**D**). Cell death was evaluated as described under "Materials and Methods". *Bars*, the mean ± SE of three independent experiments performed in triplicate. *** P<0.001 (Anova/Bonferroni), n.s., not significant.

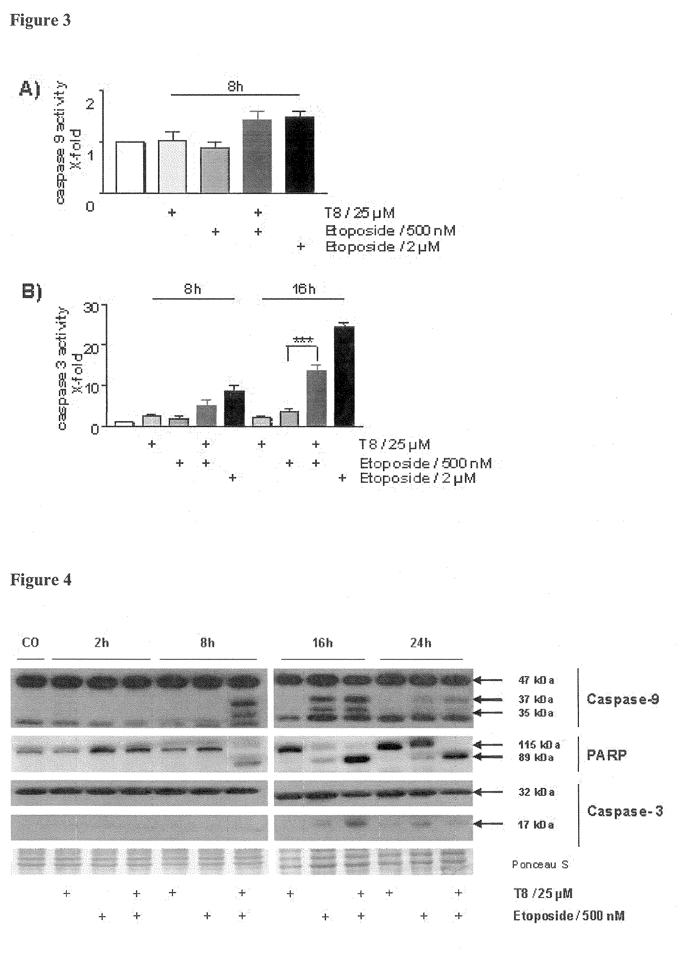

**Figure 2****:** Compound I-1 sensitizes to a reduction of etoposide-induced clonogenic survival. Wild type Jurkat cells were left untreated **(A)** or treated for 1h with 25µM of compound I-1 (**B**), 500nM etoposide (**C**) or a combination of both (**D**). After replacement into fresh methylcellulose medium, cells were incubated for 7 days. Afterwards colony number was counted (**E**). *Bars*, the mean ± SE of three independent experiments. *** P<0.001 (Anova/Bonferroni). **Figure 3****:** Compound I-1 increases initiator caspase activities. Wild type Jurkat cells were left untreated or treated with 25µM of compound I-1, 500nM etoposide or a combination of both for the indicated time periods. Fluorescence measurements were performed for caspase-9 (**A**) and caspase-3 (**B**). Protein concentration was used for normalization. *Bars*, the mean ± SE of three independent experiments performed in triplicate. *** P<0.001 (Anova/Bonferroni).

**Figure 4****:** Compound I-1 activates initiator caspases and induces PARP cleavage. Wild type Jurkat cells were left untreated or treated with 25µM of compound I-1, 500nM etoposide or a combination of both for the indicated time periods. Expression of caspase-9, caspase-3 and PARP was assessed by Western Blot analysis. Cleavage products are indicated by arrows. Ponceau S was used as loading control. A representative experiment out of three independent experiments is shown.

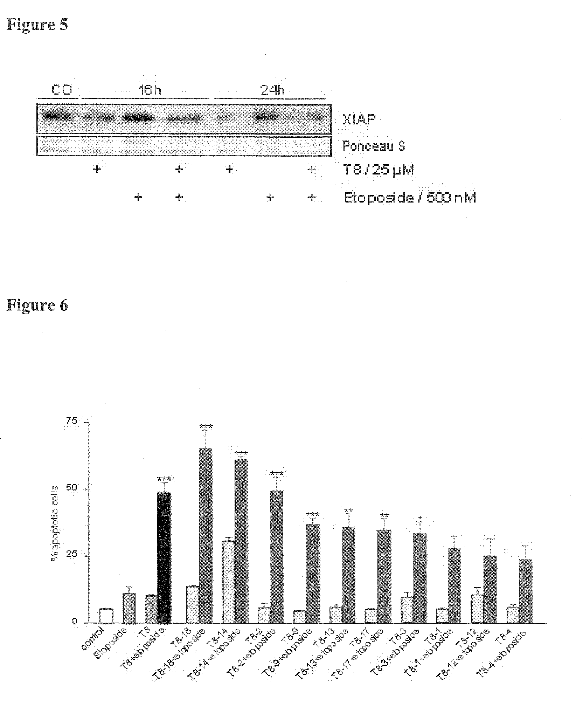

**Figure 5****:** XIAP protein level is reduced by compound I-1. Cells were left untreated (CO) or treated with 25µM of compound I-1, 500nM etoposide or with combination of both for the indicated time periods. XIAP protein level was detected by Western Blot analysis. Ponceau S was used as loading control. A representative experiment out of three independent experiments is shown.

**(****Figure 6****:** Testing of derivatives of compound I-1 for their apoptosis induction and sensitizing potential. Wild type Jurkat cells were left untreated (control) or treated with 25µM test compound, 500nM etoposide or with combination of both. Cell death was evaluated as described under "Materials and Methods". Data are the mean of three independent experiments performed in triplicate. Statistical analysis was performed with ANOVA with Bonferroni multiple comparison post test: 500nM etoposide vs. derivatives of compound I-1 and 500nM etoposide.* p value<0,05; ** p <0,01; ***p <0,001.

### Examples

### Materials and Methods

**Test Compounds**. The test compounds were purchased from Asinex Europe B.V. (Rijswijk, The Netherlands). Before application, the compounds to be tested were dissolved and further diluted in DMSO. Final DMSO concentration did not exceed 1%, a concentration verified not to interfere with the experiments performed. Etoposide was purchased from Merck Biosciences (Darmstadt, Germany), propidium iodide (PI) from Sigma (Deisenhofen, Germany).

**Cell Culture.** Human leukemia Jurkat T cells (clone J16), Jurkat T-cells stably transfected with either empty vector (Jurkat vector, pBabe Jurkat) or XIAP (Jurkat-XIAP) as well as the BIR-3 domain only (BIR-3-sp-RING-Jurkat) (kindly provided by Dr. Colin Duckett at the University of Michigan) were cultured (37°C and 5% CO₂ in RPMI 1640 containing 2 mM L-glutamine (PAN Biotech, Aidenbach, Germany) supplemented with 10% FCS gold (PAA Laboratories, Cölbe, Germany) and 1% Pyruvat (Sigma Deisenhofen, Germany).

**Detection an Quantification of Apoptosis.** Quantification of apoptosis was performed according to Nicoletti et al. (J Immunol. Methods (1991) 139, 271-279). Briefly, cells were incubated for 24 h in a hypotonic buffer (0.1% sodium citrate, 0.1% Triton-X-100 and 50 µg/ml PI) and analyzed by flow cytometry on a FACSCalibur (Becton Dickinson, Heidelberg, Germany). Nuclei to the left of the G₁-peak containing hypodiploid DNA were considered apoptotic.

**Western Blot Analysis.** Cells were collected by centrifugation, washed with ice-cold PBS and lysed in 1% Triton X-100, 0.15 M NaCl and 30 mM Tris-HCl pH 7.5 with the protease inhibitor complete™ (Roche, Mannheim, Germany) for 30 min. Lysates were centrifuge at 10,000xg for 10 min at 4°C. Equal amounts of protein were separated by SDS-PAGE (12% for XIAP) transferred to polyvinylidene difluoride membranes (Immobilon-P™, Millipore, Eschborn, Germany). Equal protein loading was controlled by coomassie staining of gels. Membranes were blocked with 5% fat-free milk powder in PBS containing 0.05% Tween-20 (1 h) and incubated with specific antibodies against XIAP (mouse antibody IgG1, clone 28, BD Transduction Laboratories, Heidelberg, Germany) overnight at 4°C. Specific proteins were visualized by secondary antibodies conjugated to horseradish peroxidase and the ECL Plus™ Western Blotting detection reagent (Amersham Biosciences, Freiburg, Germany). Membranes were exposed to X-ray film for the appropriate time periods and subsequently developed in a tabletop film processor (Curix 60, Agfa, Cologne, Germany). Equal protein loading was controlled by Ponceau S staining of membranes.

**Clonogenic assay.** Wildtype Jurkat cells were left untreated or treated for 1h with 25µM test compound, 500nM etoposide or a combination of both. Subsequently, cells were washed with PBS and resuspended in culture medium (5x105 cells/ml). Cell suspensions were diluted 1:10 with methylcellulose (0.52%) medium containing 40% FCS. Cells were seeded in 96-well plates (100 µl) and colonies were scored after 7 days of culture.

**Caspase-9/3 activity assay.** Caspase-9/3 activity was measured using caspase-9(Ac-LEHD-AFC) or caspase 3(Ac-DEVD-AFC) substrates (both from Bachem AG, Bubendorf, Germany). Jurkat leukemia T cells were left untreated or treated with test compound, etoposide or a combination of both for the indicated time period. Cells were collected by centrifugation, washed with ice-cold PBS and lysed at -85°C in 5mM MgCl₂, 1mM EGTA, 0,1% Triton, 25mM HERPES, pH 7.5. Afterwards lysates were centrifuged (14,000 rpm, 40°C) and supernatants were incubated with caspase substrate in 96-well plates. The reading was performed in a plate-reading multifunction photometer (SPECTRAFLUOR PLUS, Tecan, Crailsheim, Germany). The activity was calculated from the difference between fluorescence in the 0 point and after 1h (for caspase-3) or after 3h (for caspase-9). Protein concentration was used for normalization.

**Statistical analysis.** All experiments were performed at least three times in triplicate. Results are expressed as mean value ± SE. One-way ANOVA with Bonferroni's post test was performed using GraphPad Prism version 3.00 for Windows (GraphPad Software, San Diego California, USA). P values < 0.05 were considered significant.

### Results

Compounds were first tested for their apoptotic potential in Jurkat cells alone as well as in combination with sub-optimal concentrations of etoposide. Table 2 shows the percentage of apoptotic cells including standard deviation. The results of the cell tests revealed a distinct apoptosis enhancing effect for compounds I-1 and II-1. Whereas 25 µM of compound I-1 and 0.75 µM etoposide, respectively, showed some apoptotic effects themselves, their combination strongly induced cell death (65.7% apoptotic cells). Similar results were obtained for compound II-1 (55.8% apoptotic cells).

**Table 1: Percentage of apoptotic Jurkat cells measured after application of medium control, 0.75 µM etoposide, 25 µM test substance, and a mixture of 0.75 µM etoposide and 25 µM test substance, respectively.^{a}**

| Internal number | Control (DMSO) | Etoposide 0.75µM | Substance 25µM | Etoposide 0.75µM and Substance 25µM |
|---|---|---|---|---|
| I-1 | 9.07±1.47 | 30.66±9.89 | 13.16±2.28 | 65.74±6.0 |
| II-1 | 7.25±2.29 | 31.31±2.74 | 10.69±1.25 | 55.82±12.23 |

| | | | | |
|---|---|---|---|---|
| ^{a} mean value and standard deviation (C.I.₉₅); *n* = 3 | | | | |

In a further step Jurkat cells either overexpressing the full length XIAP gene or the smaller BIR3 domain were used to gain insights on the mechanism of action of the compounds of the present invention. Compound I-1 was tested alone or with low dose of etoposide in wild type Jurkat cells and Jurkat cells overexpressing full-length XIAP or BIR-3/Ring. Clearly compound I-1 potentiates apoptosis induced by subtoxic concentrations of etoposide (Figure 1).

Compound I-1 does not only sensitize etoposide-treated cells for apoptotic cells death, it also sensitizes for reduction of clonogenic survival of etoposide treated Jurkat cells (Figure 2).

Caspase activities induced by etoposide are significantly increased by compound I-1 (Figure 3). Since caspase-3 is activated by caspase-9 which is targeted by the BIR-3 domain, compound I-1 not only potentiates etoposide induced caspase-9 but also the downstream effector caspase-3 activity.

Figure 4 confirms the caspase-activity data by showing the strong impact of compound I-1 on cleavage of caspase-9 and -3 proforms as well as PARP the substrate of caspase-3 induced by etoposide in a time dependent manner.

Figure 5 shows another interesting feature of compound I-1. Compound I-1 alone as well as in combination with etoposide is able to reduce XIAP protein after 16 and 24 h.

In addition to compound I-1, derivatives thereof were investigated.

**Table 2: Derivatives of compound I-1.**

| | | | | | | | | |
|---|---|---|---|---|---|---|---|---|
| 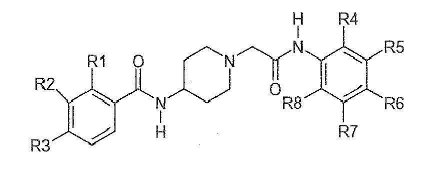 | | | | | | | | |

| Compound | R1 | R2 | R3 | R4 | R5 | R6 | R7 | R8 |
|---|---|---|---|---|---|---|---|---|
| I-1 | H | F | H | Et | H | H | H | Et |
| I-2 | H | F | H | Me | H | H | Me | H |
| I-3 | Br | H | H | Et | H | H | H | Et |
| I-4 | H | F | H | H | Me | H | Me | H |
| 1-5 | Br | H | H | Et | H | H | H | H |
| I-6 | H | H | Me | Me | H | Me | H | Me |
| I-7 | Me | H | H | H | Cl | H | H | H |
| I-8 | H | OMe | H | Me | H | Me | H | Me |
| I-9 | H | F | H | OEt | H | H | H | OEt |
| I-10 | | | | | I-11 | | | |
| 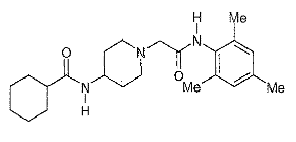 | | | | | 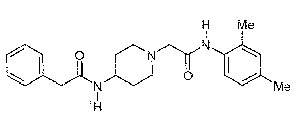 | | | |

Derivatives of compound I-1 were tested for apoptosis-induction. Compounds were either tested alone or in combination with suboptimal concentration of etoposide. As shown in Figure 6 a combination of the present compounds and etoposide significantly increases the percentage of apoptotic cells as compared to etoposide alone and the effect is clearly more than additive. In addition, compounds falling within the scope of the present invention can induce apoptosis themselves suggesting additional targets. Without wishing to be bound by theory, these results indicate that inhibition of XIAP using the present compounds may overcome the protective effect of XIAP protein to cells and, therefore, sensitizes cells to etoposide induced apoptosis.

Without wishing to be bound by theory, the above pharmacological data indicates XIAP as a potential target upon which the apoptotic effects of the present compounds may be based.

## Claims

1. A compound of formula (I) 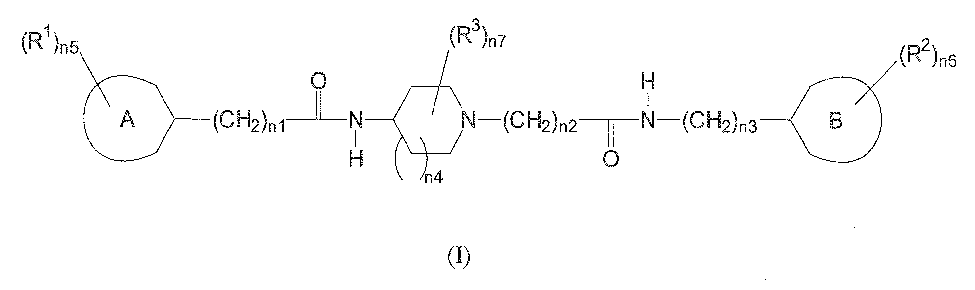 wherein
A is a saturated, unsaturated or aromatic 5 to 10-membered mono- or bicyclic ring system, which optionally contains one or more heteroatoms, selected from N, O and S;
B is a saturated, unsaturated or aromatic 5 to 10-membered mono- or bicyclic ring system, which optionally contains one or more heteroatoms, selected from N, O and S; each R¹ is independently selected from C₁₋₄ alkyl, C₁₋₄ alkoxy, C₆₋₁₀ aryl, SO₂NH-C₁₋₄ alkyl, SO₂NH-C₆₋₁₀ aryl and halogen; each R² is independently selected from C₁₋₄ alkyl, C₁₋₄ alkoxy, C₆₋₁₀ aryl, SO₂NH-C₁₋₄ alkyl, SO₂NH-C₆₋₁₀ aryl and halogen; each R³ is independently selected from C₁₋₄ alkyl, C₁₋₄ alkoxy, C₆₋₁₀ aryl, SO₂NH-C₁₋₄ alkyl, SO₂NH-C₆₋₁₀ aryl and halogen, or wherein two R³, which are bonded to neighboring carbon atoms, can be combined to form, together with the carbon atoms to which they are bonded, a fused 6-membered aromatic ring;
n1 is an integer from 0 to 3;
n2 is an integer from 0 to 3;
n3 is an integer from 0 to 3;
n4 is an integer from 0 to 2;
n5 is an integer from 0 to 3;
n6 is an integer from 0 to 5; and
n7 is an integer from 0 to 3,
wherein in each aryl group one or more carbon atoms may optionally be replaced by a heteroatom selected from N, O and S,
or a pharmaceutically acceptable salt thereof, a pharmaceutically acceptable solvate thereof or a pharmaceutically acceptable prodrug thereof,
for use as a medicament.

2. The compound of claim 1, wherein A is a saturated or aromatic 6-membered carbocyclic ring.

3. The compound of claim 1 or 2, wherein B is an aromatic 6-membered carbocyclic ring.

4. The compound of any of claims 1 to 3, wherein each R¹ is independently selected from C₁₋₄ alkyl, C₁₋₄ alkoxy and halogen.

5. The compound of any of claims 1 to 4, wherein each R² is independently selected from C₁₋₄ alkyl, C₁₋₄ alkoxy and halogen.

6. The compound of any of claims 1 to 5, wherein n1 is 0 or 1.

7. The compound of any of claims 1 to 6, wherein n2 is 1.

8. The compound of any of claims 1 to 7, wherein n3 is 0.

9. The compound of any of claims 1 to 8, wherein n4 is 1.

10. The compound of any of claims 1 to 9, wherein n7 is 0.

11. The compound of claim 1, wherein the compound of formula (I) has one of the following formulae (I-1) to (I-11): 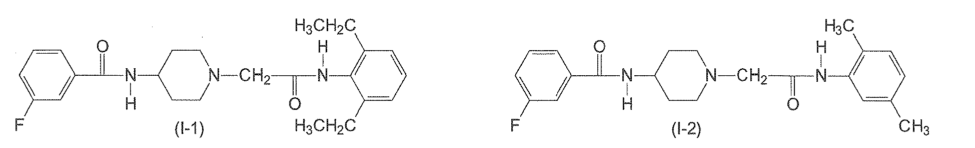 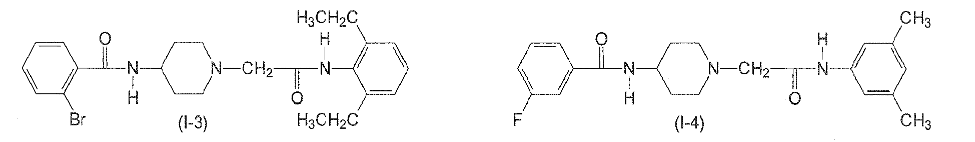 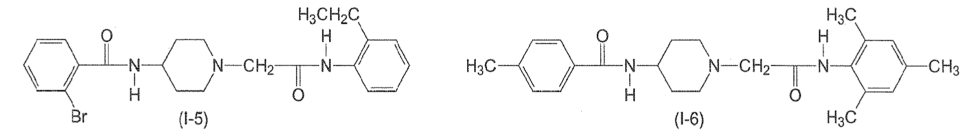 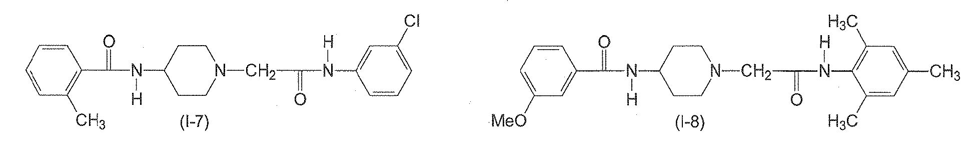 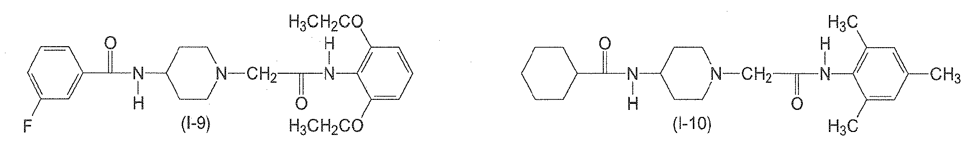 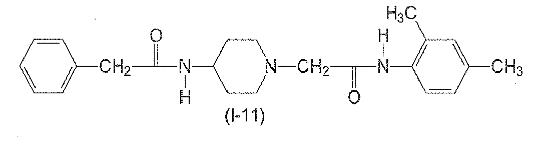 or a pharmaceutically acceptable salt thereof, a pharmaceutically acceptable solvate thereof or a pharmaceutically acceptable prodrug thereof.

12. A compound of formula (II) 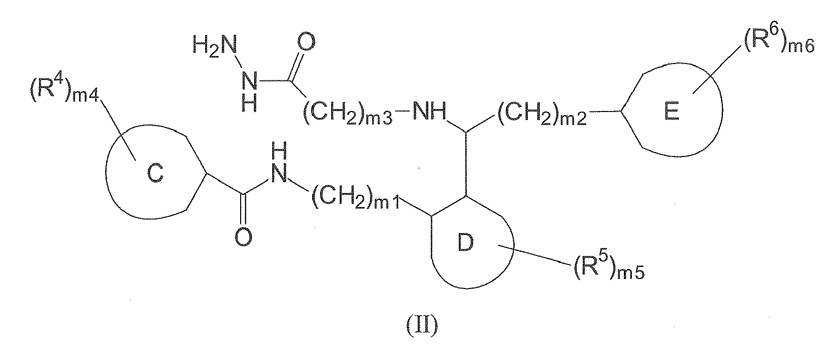 wherein
C is a saturated, unsaturated or aromatic 5 to 10-membered mono- or bicyclic ring system, which optionally contains one or more heteroatoms, selected from N, O and S;
D is a saturated, unsaturated or aromatic 5 to 10-membered mono- or bicyclic ring system, which optionally contains one or more heteroatoms, selected from N, O and S;
E is a saturated, unsaturated or aromatic 5 to 10-membered mono- or bicyclic ring system, which optionally contains one or more heteroatoms, selected from N, O and S; each R⁴ is independently selected from C₁₋₄ alkyl, C₁₋₄ alkoxy, C₆₋₁₀ aryl, SO₂NH-C₁₋₄ alkyl, SO₂NH-C₆₋₁₀ aryl and halogen; each R⁵ is independently selected from C₁₋₄ alkyl, C₁₋₄ alkoxy, C₆₋₁₀ aryl, SO₂NH-C₁₋₄ alkyl, SO₂NH-C₆₋₁₀ aryl and halogen; each R⁶ is independently selected from C₁₋₄ alkyl, C₁₋₄ alkoxy, C₆₋₁₀ aryl, SO₂NH-C₁₋₄ alkyl, SO₂NH-C₆₋₁₀) aryl and halogen; m1 is an integer from 0 to 2;
m2 is an integer from 0 to 2;
m3 is an integer from 1 to 3;
m4 is an integer from 0 to 2;
m5 is an integer from 0 to 3; and
m6 is an integer from 0 to 3;
wherein in each aryl group one or more carbon atoms may optionally be replaced by a heteroatom selected from N, O and S,
or a pharmaceutically acceptable salt thereof, a pharmaceutically acceptable solvate thereof or a pharmaceutically acceptable prodrug thereof, for use as a medicament.

13. The compound of claim 12, wherein C is an aromatic 6-membered carbocyclic ring.

14. The compound of claim 12 or 13, wherein D is an aromatic 6-membered carbocyclic ring.

15. The compound of any of claims 12 to 14, wherein E is an aromatic 6-membered carbocyclic ring.

16. The compound of any of claims 12 to 15, wherein each R⁴ is independently selected from C₁₋₄ alkyl, C₁₋₄ alkoxy and halogen.

17. The compound of any of claims 12 to 16, wherein each R⁵ is independently selected from C₁₋₄ alkyl, C₁₋₄ alkoxy and halogen.

18. The compound of any of claims 12 to 17, wherein each R⁶ is independently selected from C₁₋₄ alkyl, C₁₋₄ alkoxy and halogen.

19. The compound of any of claims 12 to 18, wherein m1 is 0.

20. The compound of any of claims 12 to 19, wherein m2 is 0.

21. The compound of any of claims 12 to 20, wherein m3 is 1.

22. The compound of claim 12, wherein the compound of formula (II) has the following formula (II-1): 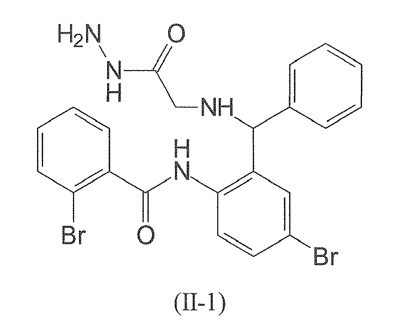 or a pharmaceutically acceptable salt thereof, a pharmaceutically acceptable solvate thereof or a pharmaceutically acceptable prodrug thereof,
for use as a medicament.

23. The compound of any of claims 1 to 22 for treating a hyperproliferative disease.

24. The compound of claim 23, wherein the hyperproliferative disease is a tumorous disease, a non-solid cancer and/or metastases.

25. The compound of claim 24, wherein the non-solid cancer is leukaemia or a lymphoid cancer.

26. The compound of claim 25, wherein the lymphoid cancer is a B-cell malignancy.

27. The compound of claim 26, wherein the B-cell malignancy is B-cell lymphoma, non-Hodgkin lymphoma or B-cell derived chronic lymphatic leukemia (B-CLL).

28. The compound of claim 23, wherein the hyperproliferative disease is hyperplasis, fibrosis, angiogenesis, psoriasis, atherosclerosis or smooth muscle proliferation in the blood vessels.

29. The compound of any of claims 1 to 22 for treating B-cell mediated autoimmune diseases.

30. The compound of claim 29, wherein the B-cell mediated autoimmune disease is Myasthenia gravis, Morbus Basedow, Hashimoto thyreoiditis or Goodpasture syndrome.

31. The compound of any of claims 1 to 28, which is to be administered in combination with an anticancer drug or radiotherapy.

32. The compound of claim 31, wherein the anticancer drug is etoposide, taxol or doxorubicine.
